# EUROPEAN PATENT SPECIFICATION

(11) **EP 3 887 820 B1**
(45) Date of publication and mention of the grant of the patent: **07.06.2023**
(21) Application number: 19835718.8
(22) Date of filing: 26.11.2019
(51) Int. Cl.: G01N 33/02, A47J 42/00, A47J 43/04, A47J 43/07

(54) **PORTABLE DEVICE AND METHOD OF OPERATION THEREOF FOR ANALYZING SALT CONTENT IN FOOD**
TRAGBARE VORRICHTUNG UND VERFAHREN ZU DEREN BETRIEB ZUR ANALYSE DES SALZGEHALTS IN LEBENSMITTELN
DISPOSITIF PORTABLE ET PROCÉDÉ DE FONCTIONNEMENT ASSOCIÉ POUR ANALYSER UN CONTENU DE SEL DANS UN ALIMENT

(30) Priority: 26.11.2018 PT 2018115173
(43) Date of publication of application: 06.10.2021
(73) Proprietor: Universidade do Porto, 4099-002 Porto (PT); Evoleo Technologies, LDA, 4425-656 Maia (PT)
(72) Inventor: DA SILVA GONÇALVES, Carla Cristina, 4099-002 Porto (PT); RODRIGUES CARVALHO PENA, Maria João, 4099-002 Porto (PT); MARCOS MOREIRA, José Luís, 4099-002 Porto (PT); MAGALHÃES MENDES, Joaquim Gabriel, 4099-002 Porto (PT); COSTA ALVES, Maria Arminda, 4200-465 Porto (PT); DE CASTRO PINHO, Olívia Maria, 4099-002 Porto (PT); MAIA DA CRUZ MARTINS, Rodolfo Manuel, 4425-656 Maia (PT)
(74) Representative: Patentree
(86) International application number: PCT/IB2019/060170
(87) International publication number: WO 2020/109993

(56) References cited:
- EP-A1- 3 251 567
- WO-A1-2015/138961
- US-B1- 10 022 008

## Description

### TECHNICAL FIELD

This description relates to a portable, preferably automatic, device and operating method thereof for analyzing the salt content in food.

### BACKGROUND

Reducing salt intake has been a priority of public health interventions and recommendations from national and international health organizations. Reducing salt intake by decreasing salt supply through food processed by the food industry and catering meals is on the political agenda due to the high evidence of great public and economic benefit and cost-effectiveness of interventions. This portable device for the acquisition and processing of data concerning salt content in food makes it easy to quickly and easily analyze the salt content in food at the collection site, with no need for transport to a laboratory. In this way it is possible to correct the salt content of the food to be supplied to consumers if necessary.

This device is of high interest to catering agents, the food industry, nutritionists, doctors, veterinarians, food inspectors and the average consumer.

The World Health Organization (WHO) recommends that salt intake be less than 5 g/day in order to reduce the risks associated with its excessive intake. The last study with population representative data on salt intake in Portugal, conducted in 2012, revealed a consumption clearly above the recommended values, with an average intake of 10.7 g/day. Several published studies have found an association between high salt intake and the development of chronic diseases including cardiovascular diseases, the leading cause of death in Portugal and worldwide. Thus, reducing salt intake is a priority on the political agenda of developed and developing countries.

In view of the above, a method for dosing sodium in food matrices is extremely useful. The reference method currently used is atomic emission spectrophotometry, and the sample preparation process for analysis is time consuming. This method also has practical limitations, such as the impossibility of obtaining immediate results and not being portable, which makes its application in the audit and inspection routine difficult. Alternatively, the sodium ion-selective electrode potentiometric method can overcome the difficulty of automation and the speed of response required, while maintaining analytical uncertainty at an acceptable level.

There are some portable devices on the market that allow the assessment of the salt content in food. Some equipment is based on conductivity measurement, however this determination is not specific for sodium content, but is only indicated for food with large amount of salt such as brines, where the existence of sodium ions in larger quantity is assumed, not being recommended for use in other food matrices.

There are also portable devices that allow coupling of a selective sodium electrode. However, they do not allow automated analysis including the preparation of the food sample to be analyzed, requiring prior steps by the user and additional equipment (e.g. scale, volume meter, grinder and stirrer) and still relegating the calculation of the result to the user.

Direct potentiometric measurement with sodium selective electrodes requires that an ionic Strength Adjustor Buffer (ISAB) solution be added to the sample to be analyzed, in order to maintain constant ionic strength on most electrodes on the market. This step increases the complexity and duration of the analysis. However, the present device uses a selective sodium electrode that incorporates an internal reference not requiring the addition of ISAB solution.

If a selective sodium electrode requiring the addition of ISAB solution is used, it is necessary to provide the reservoir with an additional ISAB solution container, another injection pump and provide for the same amount of ISAB solution to be added to all samples before measurement.

Portable equipment for the assessment of sodium content in food is of maximum use in the area of food safety and quality. Also, the fact that in many countries, such as Portugal, legislation is in force regulating the salt content in bread, it is important that the assessment of salt content in bread is quick and easy so as to allow assessing and monitoring law compliance.

Given the importance of salt consumption reduction strategies, a Working Group led by the Directorate-General for Health was set up in 2015 in Portugal, which proposed new targets for reducing salt content in both food processed by food industries (4% reduction per year in various food categories) or in catering (reduction of salt content in soup and dish up to the reference value of 0.2g salt/100g food). Again, quickly and easily assessing salt content in food will allow to assess and monitor compliance with these targets.

EP3,251,567 discloses a food blender having a container into which food may be placed, and a cutting or mixing blade adjacent the bottom of the container normally rotated about an axis X by a rotating drive, said blade being connected to the rotating drive though a resilient flexible member allowing the blade to flex away from rotation about the axis to achieve a required processing result.

WO2015/138961 discloses a food preparation appliance that includes a base having a motor and an adaptor, a weight sensor coupled to the adaptor, and a communication component. The adaptor is configured to support a food preparation accessory. The weight sensor is configured to detect a weight applied to the adaptor. The communication component is configured to transmit data associated with a weight detected by the weight sensor. An example remote communication device includes a display device, a processor; and a memory coupled to the processor. The remote communication device is configured to display a first instruction on the display device, the first instruction including a first target weight of a first substance; receive a measurement from a food preparation appliance indicative of a weight applied to an adaptor of the food preparation appliance; and display a second instruction when the weight is substantially equal to the first target weight.

US10,022,008 discloses a cooking assistive device comprising a portable housing configured to be disposed on or proximate a food item. The device includes a plurality of sensors situated within the housing, a dispenser, and a utensil holder. The device has a memory storing computer-readable instructions, and a processor. The processor is configured to execute the instructions to: (a) obtain a wireless input from a mobile device; (b) access data stored in a remote database; (c) obtain a reading from at least one of the plurality of sensors; and (d) cause the dispenser to dispense a substance in response to the reading.

These facts are described in order to illustrate the technical problems solved by the embodiments of the present invention.

### GENERAL DESCRIPTION

The invention is defined by a portable device and method for analyzing the salt content in food, as delimited in independent claims 1 and 10.

This disclosure comprises a portable multifunctional device that enables automatic preparation of solid and liquid food samples, determination of the salt content thereof through the direct sodium ion-selective electrode potentiometric method and nutritional information.

In particular, the system allows the determination of the salt and sodium content in food, expressed in g/100g (or mg/100g), the classification of salt content in associated classes, based on reference values and their association with colors (e.g. in the form of a traffic light) and still an indication of the percentage contribution of a given food to the total maximum intake recommended for adults.

Measurement of salt content with the portable device is preferably performed as follows: i) after selecting the food category on the touch screen, the device automatically starts calibration with measurement of a standard sodium chloride solution in the sample preparation unit; (ii) the electrode and the cup of the sample preparation unit are then washed, and the sample is introduced and grinding is performed together with deionized water; (iii) the equipment reads the sodium value and the temperature of the standard solution and the sample by calculating, based on Nernst's law, the salt and sodium concentration, which are displayed on the device screen, enabling it to be exported (interface with laptop, tablet, smartphone).

Expression of results is performed in such a way as to be readily and easily interpreted by the user, whereupon in addition to the salt and sodium content in g/100g (or mg/100g) and the contribution of the food according to the recommended maximum daily intake defined by the WHO, the device also preferably provides interpretative color information as a nutritional traffic light (red, yellow and green) indicating whether the salt content is adequate or excessive compared to the recommended maximum daily intake defined by the WHO.

This device is useful for quickly assessing salt content in food and in the environment of cooking the food, or where it is served, while providing relevant and unique nutritional information as it integrates the contribution of the various types of food in a meal, which is particularly important in the areas of nutrition, catering, veterinary medicine, medicine, and also in general health.

A possible embodiment of the portable automatic device for determining the salt content of solid and liquid food samples is characterized in that it has:
load cells (1) for determining the sample weight,
level sensor of the sample in the cup (2) for volume gauging,
salt sensor (3) by sodium ion-selective ion electrode,
temperature sensor (4),
motor for controlling the grinding and stirring process of the sample (6),
a pump which allows adding water (7) and a pump for adding a calibration standard (8)
touch screen (10),
Wi-Fi connection (9).

One embodiment is characterized by using an electrode that combines two electrodes, a reference electrode and a sodium ion-selective electrode.

One embodiment is characterized by performing salt content measurement by direct potentiometry using the Nernst equation as the theoretical quantification model.

One embodiment is characterized by performing salt content measurement of the food after automatic calibration of the device with only a standard solution with known sodium concentration.

One embodiment is characterized by the automation of the weighing process of the sample to be analyzed.

One embodiment is characterized by the automation of the grinding process of the sample to be analyzed.

One embodiment is characterized by the automation of the dilution process of the sample to be analyzed with deionized water.

One embodiment is characterized by the automation of the process of gauging the volume of the sample diluted with deionized water.

One embodiment is characterized by providing information regarding the salt content in solid and liquid food after 10% dilution with deionized water.

One embodiment is characterized by allowing the correction of the effect of temperature on the reading of the electrode potential difference.

One embodiment is characterized by using a control and processing unit with touch screen (10) for inputting and displaying information and interfacing with a laptop, tablet or smartphone for exporting results.

One embodiment is characterized in that the outer covering is resistant to falls and high temperature and humidity.

One embodiment is characterized by being light and easy to carry (less than 5 kg with respective accessories and solutions, in particular less than 2 kg).

One embodiment is characterized by presenting the data in the form of: salt and sodium content in g/100g (or mg/100g), classification of salt content according to the color traffic light by reference values and indication of the percentage contribution of that food to the maximum recommended total intake for adults and children.

One embodiment is characterized by being able to synchronize with a database with information on the salt content levels of the commercially available food and also the analyzed food.

One embodiment is characterized by having the ability to make data available for aggregation in a central database, ensuring data protection and anonymity, for macro analysis of results, for example for geographic and demographic analysis.

A portable device for analyzing the salt content in food is described comprising:
a container with a mixer-grinder propeller, an electrode, motor means for driving said propeller, a weight sensor of said container, an electronic data processor, and a lid for said container;
wherein said lid comprises a water injector, a calibration solution injector, and a detachable coupling on the lid for the electrode,
wherein said electronic data processor is configured to:
   detect the introduction of a food sample into said container by the weight gain measured by the weight sensor;
   drive the water injector to introduce water into the container up to a predetermined volume of water and drive said motor means so as to grind the contents of the container by said propeller;
   drive said motor means to stir the contents of the container by said propeller and to measure the salt concentration indicated by the electrode.

In one embodiment, said lid is coupled to said container by means of a hinged coupling.

In one embodiment, said electronic data processor, in order to initialize the salt content analyzing device, is further configured to:
drive the calibration solution injector for introducing a predetermined salt concentration calibration solution into the container and drive said motor means to stir the contents of the container;
measure the salt concentration indicated by the electrode;
calculate a calibration of said electrode as a function of the measured salt concentration and the predetermined salt concentration of the calibration solution.

In one embodiment, said electronic data processor is further configured to, upon driving said motor means for grinding the contents of the container, introduce a predetermined volume of water in the container:
previously drive the water injector to introduce a first part of the predetermined volume of water into the container before driving said motor means; and
subsequently drive the water injector to introduce a second part of the predetermined volume of water into the container after driving said motor means.

In one embodiment, said electronic data processor is further configured to drive said motor means to stir the contents of the container by said propeller and then measure the salt concentration indicated by the electrode.

In one embodiment, said electronic data processor is further configured to, upon driving the calibration solution injector to introduce the calibration solution into the container, drive the water injector to introduce water into the container to dilute the concentration of the calibration solution up to a predetermined salt concentration of the calibration solution.

In one embodiment, said electronic data processor is further configured to query a database of reference products and, depending on the measured salt concentration, display a ranking or variable color indicator with the measured salt concentration (see Department of Health, Food Standards Agency (2016). Llywodraeth Cymru Welsh Government, Food Standards Scotland, Inbhe Bìdh Alba. Guide to creating a front of pack nutrition label for pre-packed products sold through retail outlets, Food Standards Agency).

In one embodiment, said electronic data processor is further configured not to drive the water injector to introduce water into the container until it detects that the lid is closed.

In one embodiment, said electronic data processor is further configured not to drive the calibration solution injector to introduce water into the container until it detects that the lid is closed.

In one embodiment, said electronic data processor is further configured not to drive said motor means until it detects that the lid is closed.

In one embodiment, said electronic data processor is further configured not to drive the motor means for grinding until it detects that the electrode is not coupled to the lid.

In one embodiment, said electronic data processor is further configured not to drive the motor means for stirring until it detects that the electrode is coupled to the lid.

In one embodiment, the predetermined volume of water is approximately 200 mL.

In one embodiment, the water is deionized water.

One embodiment comprises a temperature sensor in said container or lid for gauging said salt concentration measurement by the electrode.

These facts are described in order to illustrate the solution or solutions to said technical problems.

### BRIEF DESCRIPTION OF THE DRAWINGS

For an easier understanding, figures are herein attached, which represent preferred embodiments which are not intended to limit the object of the present description.
**Figure 1****-** Block diagram of an embodiment of the portable device.
**Figure 2** - Representative scheme of the main components of an embodiment of the portable device in two alternative versions.
**Figure 3** - Exemplary scheme of an embodiment of the salt content measurement procedure with an embodiment of the portable device.

### DETAILED DESCRIPTION

The disclosure comprises a portable device that enables automatic preparation of solid and liquid food samples and determination of the salt content thereof through the direct potentiometric method.

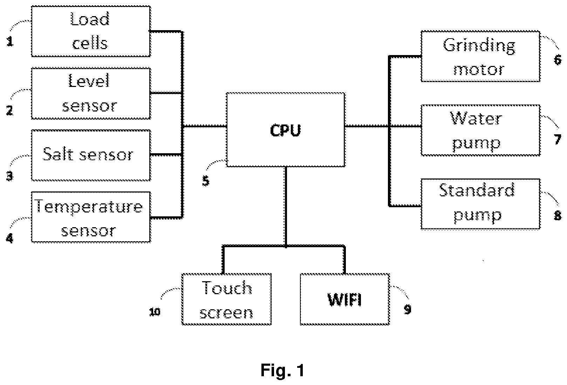

According to Figure 1, one embodiment of the device comprises a processing and control unit (5) which interconnects with sensors: load cells (1) for determining sample weight, level sensor of the sample in the cup (2) for volume gauging, salt sensor (3) and temperature sensor (4). The device controls the grinding and stirring process of the sample through a motor (6), for the preparation of the sample a pump is used which allows adding water (7) and also a pump for adding a standard (8) for calibration. The user can control the device via a touch screen (10) and can also communicate with a central server via a Wi-Fi connection (9).

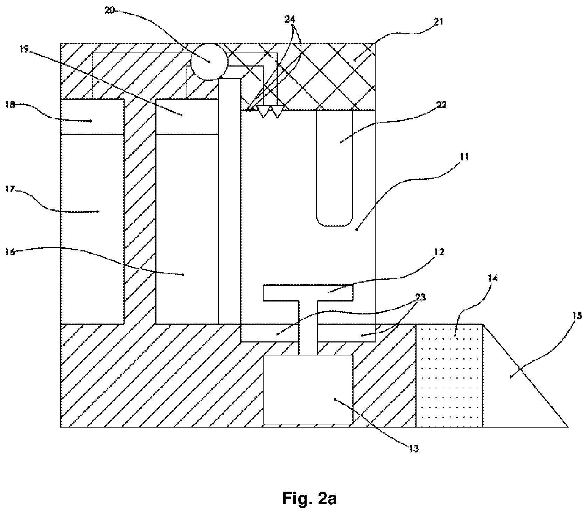

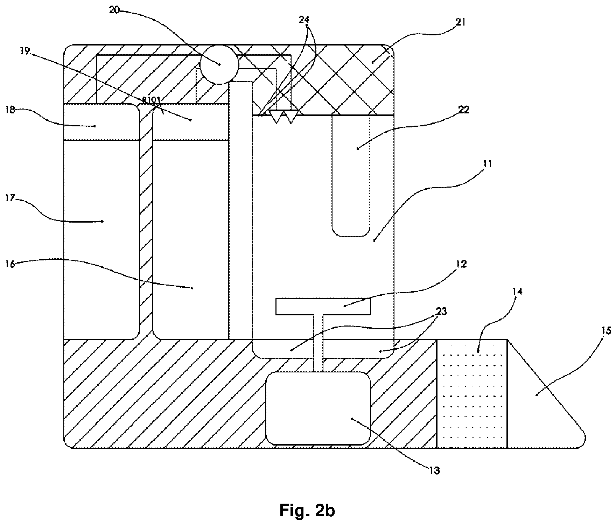

Figure 2 shows a representative scheme of the main components of a preferred embodiment of the portable device. The device consists of a cup (11) having within a grinding and stirring system (12) which grinds the sample as well as the mixture during the test. This system is driven by a motor (13) which is mounted on the structure below the cup. The cup seats on a load cell system (23) which measures the weight of the assembly. At the front part there is a CPU (14) which controls the entire system, added to it is the screen (15) which interfaces with the user. At the rear part of the structure are the deionized water (17) and standard liquid (18) tanks which in turn are connected to the deionized water (18) and standard liquid (19) pumps. These pumps feed the deionized water and standard liquid injectors (24) into the cup. The injectors are mounted on the cup lid (21) which supports the sensor (22) and also seals to the outside of the cup. The lid has a pivoting and hinged movement created by the lid rotation mechanism (20).

The control and processing unit also allows controlling the injection pumps, the grinding and stirring motor and the recording of scale and level sensor data. This unit also calculates the salt and sodium content of the sample through the data obtained: temperature and potential difference of the calibration standard and weight, dilution, temperature and potential difference of the sample.

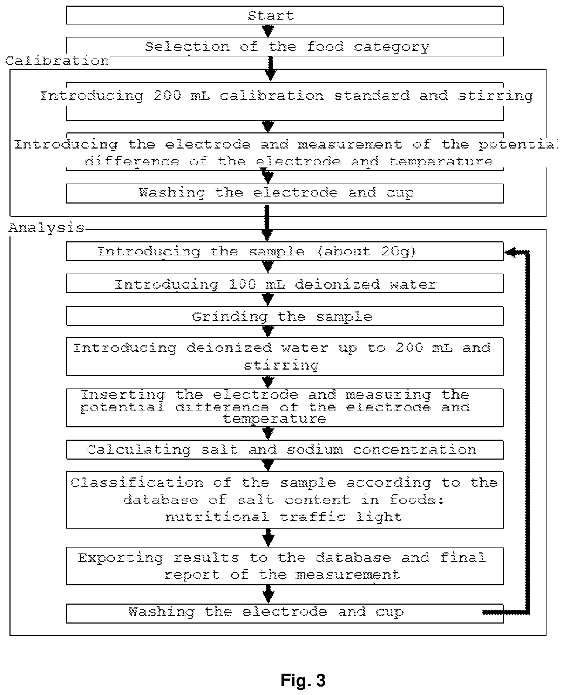

Salt content measurement with the portable device is performed according to the exemplary scheme shown in Figure 3: after selecting the food category on the touch screen (10), the device automatically starts calibration with injection of the standard solution into the sample preparation unit, with the sodium ion-selective electrode (13) inserted; stirring of the solution begins; after 3 minutes the device records the electrode potential difference measured in the solution as well as its temperature; the electrode and the sample preparation unit are removed and washed with deionized water.

In order to perform an analysis, the food to be assessed is added to the cup in the sufficient amount that is indicated by a light signal on the screen (assessed by the load cells, about 20 grams) (1); the sample weight is recorded; the device adds 100 mL of deionized water and grinds the sample; the device then completes the dilution with deionized water (up to 200 mL volume, as assessed by the level sensor); the electrode is reintroduced into the solution and stirring thereof is started; after 3 minutes the device records the potential difference of the measurement electrode inserted in the solution as well as its temperature; the control and processing unit calculates the salt and sodium concentration, which are displayed on the screen, its exporting being possible (interface with a laptop, tablet, smartphone).

The system allows the determination of salt and sodium content in food expressed in g/100g (or mg/100g) respectively, the classification of the salt content according to the color traffic light based on the reference values and also indication of the percentage of food contribution to the total maximum recommended intake for adults and children according to developed software.

The system will be interconnected to a database for the synchronization of information on salt content of different foods, allowing a comparison to be drawn between the measurements with other previous measurements.

The system allows the aggregation of information from the results of measurements made in a database, ensuring the protection and anonymity of the information in order to enable macro analysis, for example of geographic and demographic nature.

The term "comprises" or "comprising" when used herein is intended to indicate the presence of the features, elements, integers, steps and components mentioned, but does not preclude the presence or addition of one or more other features, elements, integers, steps and components, or groups thereof.

The embodiments described are combinable with each other. The present invention is of course in no way restricted to the embodiments described herein but by the subject-matter of the appended claims.

## Claims

1. Portable device for analyzing the salt content in food, comprising:
a container with a mixer-grinder propeller, a detachable electrode (13), motor means (6) for driving said propeller, a weight sensor of said container, an electronic data processor (5, 14), and a lid (21) for said container;
wherein said lid (21) comprises a water injector (24) and a calibration solution injector (24),
wherein said electronic data processor is configured to:
detect the introduction of a food sample into said container by the weight gain measured by the weight sensor;
drive the water injector (24) to introduce water (17) into the container up to a predetermined volume of water (17) and drive said motor means (6) to grind the contents of the container by said propeller;
drive said motor means (6) to stir the contents of the container by said propeller and to measure the salt concentration indicated by the electrode (13).

2. Portable device for analyzing the salt content in food according to the preceding claim, wherein the lid (21) comprises a detachable coupling on the lid (21) for coupling said electrode (13).

3. Portable device for analyzing the salt content in food according to any one of the preceding claims, wherein said lid (21) is coupled to said container by means of a hinged coupling, in particular comprising a temperature sensor (4) in said container or lid (21) for gauging the measurement by the electrode of salt concentration.

4. Portable device for analyzing the salt content in food according to any one of the preceding claims, wherein said electronic data processor (5, 14) for initializing the device for analyzing salt content is further configured to:
drive the calibration solution injector (24) for introducing a predetermined salt concentration calibration solution into the container and driving said motor means (6) to stir the contents of the container;
measure the salt concentration indicated by the electrode (13);
calculate a calibration of said electrode (13) as a function of the measured salt concentration and the predetermined salt concentration of the calibration solution.

5. Portable device for analyzing the salt content in food according to any one of the preceding claims, wherein said electronic data processor (5, 14) is further configured to, upon driving said motor means (6) for grinding the contents of the container, introduce a predetermined volume of water in the container:
previously drive the water injector (24) to introduce a first part of the predetermined volume of water (17) into the container before driving said motor means (6); and
subsequently drive the water injector (24) to introduce a second part of the predetermined volume of water (17) into the container after driving said motor means (6).

6. Portable device for analyzing the salt content in food according to any one of the preceding claims, wherein said electronic data processor (5, 14) is further configured to:
drive said motor means (6) to stir the contents of the container by said propeller and to subsequently measure the salt concentration indicated by the electrode (13).

7. Portable device for analyzing the salt content in food according to any one of the preceding claims, wherein said electronic data processor (5, 14) is further configured to:
when driving the calibration solution injector (24) to introduce the calibration solution into the container, drive the water injector (24) to introduce water (17) into the container to dilute the calibration solution concentration up to a predetermined salt concentration of the calibration solution.

8. Portable device for analyzing the salt content in food according to any one of the preceding claims, comprising a screen (10, 15) and wherein said electronic data processor (5, 14) is further configured to:
query a database of reference products and, depending on the measured salt concentration, display a ranking;
or display a variable color indicator on the screen (10, 15) with the measured salt concentration.

9. Portable device for analyzing the salt content in food according to any one of the preceding claims, wherein said electronic data processor (5, 14) is further configured not to drive the motor means (6) for grinding until it detects that the electrode (13) is not coupled to the lid (21); or
wherein said electronic data processor (5, 14) is further configured not to drive the motor means (6) for stirring until it detects that the electrode (13) is coupled to the lid (21); or
said portable device comprising a closure detector of said lid (21) and wherein said electronic data processor (5, 14) is further configured:
not to drive the water injector (24) to introduce water (17) into the container until it detects that the lid (21) is closed; or
wherein said electronic data processor (5, 14) is further configured not to drive the calibration solution injector (24) to introduce calibration solution into the container until it detects that the lid (21) is closed; or
wherein said electronic data processor (5, 14) is further configured not to drive said motor means (6) until it detects that the lid (21) is closed.

10. Method of operating a portable device for analyzing the salt content in food, comprising:
providing a device comprising a container with a mixer-grinder propeller, a detachable electrode (13), motor means (6) for driving said propeller, a weight sensor of said container, an electronic data processor (5, 14), and a lid (21) for said container; wherein said lid (21) comprises a water injector (24) and a calibration solution injector (24);
detecting the introduction of a food sample into said container by the weight gain measured by the weight sensor;
driving the water injector (24) to introduce water (17) into the container up to a predetermined volume of water (17) and drive said motor means to grind the contents of the container by said propeller;
driving said motor means (6) to stir the contents of the container by said propeller and to measure the salt concentration indicated by the electrode (13).

11. Method of operating a portable device for analyzing the salt content in food according to claim 10, comprising, for initializing the device for analyzing the salt content:
driving the calibration solution injector (24) for introducing a predetermined salt concentration calibration solution into the container and driving said motor means to stir the contents of the container;
measuring the salt concentration indicated by the electrode;
calculating a calibration of said electrode as a function of the measured salt concentration and the predetermined salt concentration of the calibration solution;
in particular, when driving the calibration solution injector (24) to introduce the calibration solution into the container, operating the water injector to introduce water into the container to dilute the calibration solution concentration up to a predetermined salt concentration of the calibration solution.

12. Method of operating a portable device for analyzing the salt content in food according to claim 10 or 11, which, by driving said motor means (6) to grind the contents of the container, comprises, for introducing a predetermined volume of water (17) into the container:
previously driving the water injector (24) to introduce a first part of the predetermined volume of water into the container before driving said motor means; and
subsequently driving the water injector (24) to introduce a second part of the predetermined volume of water into the container after driving said motor means.

13. Method of operating a portable device for analyzing the salt content in food according to any one of claims 10-12, comprising:
querying a database of reference products and, depending on the measured salt concentration, displaying a ranking;
or display a variable color indicator on a screen with the measured salt concentration.

14. Method of operating a portable device for analyzing the salt content in food according to any one of claims 10-13 comprising not driving the water injector to introduce water into the container until it detects that the lid is closed; or not driving the calibration solution injector to introduce solution into the container until it detects that the lid is closed; or not driving said motor means until it detects that the lid is closed; or not driving the motor means for grinding until it detects that the electrode is not coupled to the lid; or not driving the motor means for stirring until it detects that the electrode is coupled to the lid, or combinations thereof.

15. Method of operating a portable device for analyzing the salt content in food according to any one of claims 10-14 comprising accessing a database and storing the data of the measurement performed comprising the measured salt concentration.

## Patentansprüche

1. Tragbare Vorrichtung zur Analyse des Salzgehalts in Lebensmitteln, umfassend:
einen Behälter mit einem Propeller zum Mischen und Zerkleinern, eine abnehmbare Elektrode (13), einen Motor (6) zum Antrieb des genannten Propellers, einen Gewichtssensor des genannten Behälters, einen elektronischen Datenprozessor (5, 14) und einen Deckel (21) für den genannten Behälter;
wobei der genannte Deckel (21) einen Einspritzdüse für Wasser (24) und eine Einspritzdüse für eine Kalibrierungslösung (24) umfasst,
wobei der genannte elektronische Datenprozessor so konfiguriert ist, dass er:
das Einbringen einer Lebensmittelprobe in den genannten Behälter anhand der von dem Gewichtssensor gemessenen Gewichtszunahme erkennt;
die Einspritzdüse für Wasser (24) aktiviert, um Wasser (17) bis zu einer vorbestimmten Wassermenge (17) in den Behälter einzuspritzen, und den genannten Motor (6) aktiviert, um den Inhalt des Behälters durch den Propeller zu zerkleinern;
den genannten Motor (6) aktiviert, um den Inhalt des Behälters durch den genannten Propeller zu durchmischen und um die von der Elektrode (13) angezeigte Salzkonzentration zu messen.

2. Tragbare Vorrichtung zur Analyse des Salzgehalts in Lebensmitteln nach dem vorangehenden Anspruch, wobei der Deckel (21) eine lösbare Kupplung am Deckel (21) umfasst, um die Elektrode (13) zu befestigen.

3. Tragbare Vorrichtung zur Analyse des Salzgehalts in Lebensmitteln nach einem der vorangehenden Ansprüche, wobei der genannte Deckel (21) über eine Gelenkverbindung mit dem genannten Behälter verbunden ist, insbesondere umfassend einen Temperatursensor (4) in dem genannten Behälter oder Deckel (21) um die Messung der Salzkonzentration durch die Elektrode zu kalibrieren.

4. Tragbare Vorrichtung zur Analyse des Salzgehalts in Lebensmitteln nach einem der vorangehenden Ansprüche, wobei der genannte elektronische Datenprozessor (5, 14) zum Initialisieren der Vorrichtung zur Analyse des Salzgehalts ferner so konfiguriert ist, dass er:
die Einspritzdüse für die Kalibrierungslösung (24) aktiviert, um eine Kalibrierungslösung mit vorbestimmter Salzkonzentration in den Behälter einzuspritzen, und um den genannten Motor (6) zu aktivieren, um den Inhalt des Behälters umzurühren;
die von der Elektrode (13) angezeigte Salzkonzentration misst;
eine Kalibrierung für die genannte Elektrode (13) aus einer Funktion der gemessenen Salzkonzentration und der vorbestimmten Salzkonzentration der Kalibrierungslösung berechnet.

5. Tragbare Vorrichtung zur Analyse des Salzgehalts in Lebensmitteln nach einem der vorangehenden Ansprüche, wobei der genannte elektronische Datenprozessor (5, 14) ferner so konfiguriert ist, dass er beim Aktivieren des genannten Motors (6) zum Zerkleinern des Inhalts des Behälters eine vorbestimmte Wassermenge in den Behälter einspritzt:
zuvor die Einspritzdüse für Wasser (24) aktiviert, um einen ersten Teil der vorbestimmten Wassermenge (17) in den Behälter einzusprühen, bevor der genannte Motor (6) aktiviert wird; und
anschließend die Einspritzdüse für Wasser (24) aktiviert, um einen zweiten Teil der vorbestimmten Wassermenge (17) in den Behälter einzusprühen, nachdem der genannte Motor (6) aktiviert wurde.

6. Tragbare Vorrichtung zur Analyse des Salzgehalts in Lebensmitteln nach einem der vorangehenden Ansprüche, wobei der genannte elektronische Datenprozessor (5, 14) ferner so konfiguriert ist, dass er:
den genannten Motor (6) aktiviert, um den Inhalt des Behälters durch den genannten Propeller umzurühren und um anschließend die von der Elektrode (13) angezeigte Salzkonzentration zu messen.

7. Tragbare Vorrichtung zur Analyse des Salzgehalts in Lebensmitteln nach einem der vorangehenden Ansprüche, wobei der genannte elektronische Datenprozessor (5, 14) ferner so konfiguriert ist, dass er:
wenn er die Einspritzdüse für die Kalibrierungslösung (24) aktiviert, um die Kalibrierungslösung in den Behälter einzuspritzen, die Einspritzdüse für Wasser (24) aktiviert, um Wasser (17) in den Behälter einzuspritzen, um die konzentrierte Kalibrierungslösung bis zu einer vorbestimmten Salzkonzentration der Kalibrierungslösung zu verdünnen.

8. Tragbare Vorrichtung zur Analyse des Salzgehalts in Lebensmitteln nach einem der vorangehenden Ansprüche, umfassend einen Bildschirm (10, 15), wobei der genannte elektronische Datenprozessor (5, 14) ferner so konfiguriert ist, dass er:
eine Datenbank mit Referenzprodukten abfragt und, in Abhängigkeit von der gemessenen Salzkonzentration, eine Rangfolge anzeigt;
oder einen variablen Farbindikator mit der gemessenen Salzkonzentration auf dem Bildschirm (10, 15) anzeigt.

9. Tragbare Vorrichtung zur Analyse des Salzgehalts in Lebensmitteln nach einem der vorangehenden Ansprüche, wobei der genannte elektronische Datenprozessor (5, 14) ferner so konfiguriert ist, dass er den Motor (6) zum Zerkleinern nicht aktiviert, bis er erkennt, dass die Elektrode (13) nicht mit dem Deckel (21) verbunden ist; oder
wobei der genannte elektronische Datenprozessor (5, 14) ferner so konfiguriert ist, dass er den Motor (6) zum Umrühren nicht aktiviert, bis er erkennt, dass die Elektrode (13) mit dem Deckel (21) verbunden ist; oder
wobei die genannte tragbare Vorrichtung einen Detektor am Verschluss des genannten Deckels (21) umfasst und wobei der genannte elektronische Datenprozessor (5, 14) ferner so konfiguriert ist, dass er:
die Einspritzdüse für Wasser (24) nicht aktiviert, um Wasser (17) in den Behälter einzuspritzen, bis er erkennt, dass der Deckel (21) geschlossen ist; oder
wobei der genannte elektronische Datenprozessor (5, 14) ferner so konfiguriert ist, dass er die Einspritzdüse für die Kalibrierungslösung (24) nicht aktiviert, um die Kalibrierungslösung in den Behälter einzuspritzen, bis er erkennt, dass der Deckel (21) geschlossen ist; oder
wobei der genannte elektronische Datenprozessor (5, 14) ferner so konfiguriert ist, dass der Motor (6) nicht aktiviert wird, bis er erkennt, dass der Deckel (21) geschlossen ist.

10. Verfahren zum Betrieb einer tragbaren Vorrichtung zur Analyse des Salzgehalts in Lebensmitteln, umfassend:
Bereitstellen einer Vorrichtung, umfassend einen Behälter mit einem Propeller zum Mischen und Zerkleinern, eine abnehmbare Elektrode (13), einen Motor (6) zum Antrieb des genannten Propellers, einen Gewichtssensor des genannten Behälters, einen elektronischen Datenprozessor (5, 14) und einen Deckel (21) für den genannten Behälter; wobei der genannte Deckel (21) eine Einspritzdüse für Wasser (24) und eine Einspritzdüse für eine Kalibrierungslösung (24) umfasst;
Erfassen des Einbringens einer Lebensmittelprobe in den Behälter anhand der vom Gewichtssensor gemessenen Gewichtszunahme;
Aktivieren der Einspritzdüse für Wasser (24), um Wasser (17) bis zu einer vorbestimmten Wassermenge (17) in den Behälter einzuspritzen, und um den genannten Motor zu aktivieren, um den Inhalt des Behälters durch den Propeller zu zerkleinern;
Aktivieren des genannten Motors (6), um den Inhalt des Behälters durch den Propeller zu durchmischen und die von der Elektrode (13) angezeigte Salzkonzentration zu messen.

11. Verfahren zum Betrieb einer tragbaren Vorrichtung zur Analyse des Salzgehalts in Lebensmitteln nach Anspruch 10, zum Initialisieren der Vorrichtung zur Analyse des Salzgehalts umfassend:
Aktivieren der Einspritzdüse für eine Kalibrierungslösung (24), um eine Kalibrierungslösung mit vorbestimmter Salzkonzentration in den Behälter einzuspritzen und um den genannten Motor zu aktivieren, um den Inhalt des Behälters umzurühren;
Messen der von der Elektrode angezeigte Salzkonzentration;
Berechnen einer Kalibrierung für die genannte Elektrode aus einer Funktion der gemessenen Salzkonzentration und der vorbestimmten Salzkonzentration der Kalibrierungslösung;
insbesondere, wenn die Einspritzdüse für die Kalibrierungslösung (24) aktiviert wird, um die Kalibrierungslösung in den Behälter einzuspritzen, die Einspritzdüse für Wasser aktiviert wird, um Wasser in den Behälter einzuspritzen, um die konzentrierte Kalibrierungslösung bis zu einer vorbestimmten Salzkonzentration der Kalibrierungslösung zu verdünnen.

12. Verfahren zum Betrieb einer tragbaren Vorrichtung zur Analyse des Salzgehalts in Lebensmitteln nach Anspruch 10 oder 11, das durch Aktivieren des genannten Motors (6) zum Zerkleinern des Inhalts des Behälters und zum Einspritzen einer vorbestimmten Wassermenge (17) in den Behälter umfasst:
vorheriges Aktivieren der Einspritzdüse für Wasser (24), um einen ersten Teil der vorbestimmten Wassermenge in den Behälter einzubringen, bevor der genannte Motor aktiviert wird; und
anschließendes Aktivieren der Einspritzdüse für Wasser (24), um einen zweiten Teil der vorbestimmten Wassermenge in den Behälter einzuspritzen, nachdem der genannte Motor aktiviert wurde.

13. Verfahren zum Betrieb einer tragbaren Vorrichtung zur Analyse des Salzgehalts in Lebensmitteln nach einem der Ansprüche 10-12, umfassend:
Abfrage einer Datenbank mit Referenzprodukten und, in Abhängigkeit von der gemessenen Salzkonzentration, Anzeige einer Rangfolge;
oder Anzeige eines variablen Farbindikators mit der gemessenen Salzkonzentration auf dem Bildschirm.

14. Verfahren zum Betrieb einer tragbaren Vorrichtung zur Analyse des Salzgehalts in Lebensmitteln nach einem der Ansprüche 10-13, bei dem die Einspritzdüse für Wasser nicht aktiviert wird, um Wasser in den Behälter einzuspritzen, bis sie erkennt, dass der Deckel geschlossen ist; oder die Einspritzdüse für Kalibrierungslösung nicht aktiviert wird, um Kalibrierungslösung in den Behälter einzuspritzen, bis sie erkennt, dass der Deckel geschlossen ist; oder der genannte Motor nicht aktiviert wird, bis sie erkennt, dass der Deckel geschlossen ist; oder der genannte Motor für das Zerkleinern nicht aktiviert wird, bis sie erkennt, dass die Elektrode nicht mit dem Deckel verbunden ist; oder der Motor zum Umrühren nicht aktiviert wird, bis sie erkennt, dass die Elektrode mit dem Deckel verbunden ist, oder Kombinationen davon.

15. Verfahren zum Betrieb einer tragbaren Vorrichtung zur Analyse des Salzgehalts in Lebensmitteln nach einem der Ansprüche 10-14, umfassend den Zugriff auf eine Datenbank und Speichern der Daten der durchgeführten Messung, einschließlich der gemessenen Salzkonzentration.

## Revendications

1. Dispositif portable pour analyser un contenu de sel dans un aliment, comprenant :
un récipient avec une hélice mixeuse-broyeuse, une électrode détachable (13), des moyens moteurs (6) pour opérer ladite hélice, un capteur de poids dudit récipient, un traiteur de données électronique (5, 14), et un couvercle (21) pour ledit récipient ;
dans lequel ledit couvercle (21) comprend un injecteur d'eau (24) et un injecteur de solution de calibrage (24),
dans lequel ledit traiteur de données électronique est configuré pour :
détecter l'introduction d'un échantillon d'aliment dans ledit récipient à travers l'augmentation de poids mesurée par le capteur de poids ;
opérer l'injecteur d'eau (24) pour introduire de l'eau (17) dans le récipient jusqu'à un volume d'eau prédéterminé (17) et opérer lesdits moyens moteurs (6) pour broyer les contenus du récipient à travers ladite hélice ;
opérer lesdits moyens moteurs (6) pour mélanger les contenus du récipient à travers ladite hélice et pour mesurer la concentration de sel indiquée par l'électrode (13).

2. Dispositif portable pour analyser un contenu de sel dans un aliment selon la revendication précédente, dans lequel le couvercle (21) comprend un accouplement détachable sur le couvercle (21) pour accoupler ladite électrode (13).

3. Dispositif portable pour analyser un contenu de sel dans un aliment selon l'une quelconque des revendications précédentes, dans lequel ledit couvercle (21) est accouplé audit récipient à travers un accouplement articulé, en particulier comprenant un capteur de température (4) dans ledit récipient ou couvercle (21) pour jauger la mesure, par l'électrode, de la concentration en sel.

4. Dispositif portable pour analyser un contenu de sel dans un aliment selon l'une quelconque des revendications précédentes, dans lequel ledit traiteur de données électronique (5, 14) pour initialiser le dispositif pour analyser le contenu de sel est également configuré pour :
opérer l'injecteur de solution de calibrage (24) pour introduire une solution de calibrage à concentration de sel prédéterminée dans le récipient et opérer lesdits moyens moteurs (6) pour mélanger les contenus du récipient ;
mesurer la concentration de sel indiquée par l'électrode (13) ;
calculer un calibrage de ladite électrode (13) en tant que fonction de la concentration de sel mesurée et de la concentration de sel prédéterminée de la solution de calibrage.

5. Dispositif portable pour analyser un contenu de sel dans un aliment selon l'une quelconque des revendications précédentes, dans lequel ledit traiteur de données électronique (5, 14) est également configuré, lorsque lesdits moyens moteurs sont opérés (6) pour broyer les contenus du récipient, pour introduire un volume d'eau prédéterminé dans le récipient :
opérer préalablement l'injecteur d'eau (24) pour introduire une première partie du volume d'eau prédéterminé (17) dans le récipient avant d'opérer lesdits moyens moteurs (6) ; et
opérer par la suite l'injecteur d'eau (24) pour introduire une seconde partie du volume d'eau prédéterminé (17) dans le récipient après avoir opéré lesdits moyens moteurs (6).

6. Dispositif portable pour analyser un contenu de sel dans un aliment selon l'une quelconque des revendications précédentes, dans lequel ledit traiteur de données électronique (5, 14) est également configuré pour :
opérer lesdits moyens moteurs (6) pour mélanger les contenus du récipient à travers ladite hélice et pour mesurer, par la suite, la concentration de sel indiquée par l'électrode (13).

7. Dispositif portable pour analyser un contenu de sel dans un aliment selon l'une quelconque des revendications précédentes, dans lequel ledit traiteur de données électronique (5, 14) est également configuré pour :
lorsque l'injecteur de solution de calibrage (24) est opéré, introduire la solution de calibrage dans le récipient, opérer l'injecteur d'eau (24) pour introduire de l'eau (17) dans le récipient pour diluer la concentration de solution de calibrage jusqu'à une concentration de sel prédéterminée de la solution de calibrage.

8. Dispositif portable pour analyser un contenu de sel dans un aliment selon l'une quelconque des revendications précédentes, comprenant un écran (10, 15) et dans lequel ledit traiteur de données électronique (5, 14) est également configuré pour :
consulter une base de données de produits de référence et, en fonction de la concentration de sel mesurée, afficher un classement ;
ou afficher un indicateur de couleur variable sur l'écran (10, 15) avec la concentration de sel mesurée.

9. Dispositif portable pour analyser un contenu de sel dans un aliment selon l'une quelconque des revendications précédentes, dans lequel ledit traiteur de données électronique (5, 14) est également configuré pour ne pas opérer les moyens moteurs (6) pour broyer jusqu'à ce qu'il détecte que l'électrode (13) n'est pas accouplée au couvercle (21) ; ou
dans lequel ledit traiteur de données électronique (5, 14) est également configuré pour ne pas opérer les moyens moteurs (6) pour mélanger jusqu'à ce qu'il détecte que l'électrode (13) est accouplée au couvercle (21) ; ou
ledit dispositif portable comprenant un détecteur de fermeture dudit couvercle (21) et dans lequel ledit traiteur de données électronique (5,14) est également configuré :
pour ne pas opérer l'injecteur d'eau (24) pour introduire de l'eau (17) dans le récipient jusqu'à ce qu'il détecte que le couvercle (21) est fermé ; ou
dans lequel ledit traiteur de données électronique (5, 14) est également configuré pour ne pas opérer l'injecteur de solution de calibrage (24) pour introduire la solution de calibrage dans le récipient jusqu'à ce qu'il détecte que le couvercle (21) est fermé ; ou
dans lequel ledit traiteur de données électronique (5, 14) est également configuré pour ne pas opérer lesdits moyens moteurs (6) jusqu'à ce qu'il détecte que le couvercle (21) est fermé.

10. Procédé de fonctionnement d'un dispositif portable pour analyser un contenu de sel dans un aliment, comprenant :
fournir un dispositif comprenant un récipient avec une hélice mixeuse-broyeuse, une électrode détachable (13), des moyens moteurs (6) pour opérer ladite hélice, un capteur de poids dudit récipient, un traiteur de données électronique (5, 14), et un couvercle (21) pour ledit récipient ; dans lequel ledit couvercle (21) comprend un injecteur d'eau (24) et un injecteur de solution de calibrage (24) ;
détecter l'introduction d'un échantillon d'aliment dans ledit récipient à travers l'augmentation de poids mesurée par le capteur de poids ;
opérer l'injecteur d'eau (24) pour introduire de l'eau (17) dans le récipient jusqu'à un volume d'eau prédéterminé (17) et opérer lesdits moyens moteurs pour broyer les contenus du récipient à travers ladite hélice ;
opérer lesdits moyens moteurs (6) pour mélanger les contenus du récipient à travers ladite hélice et pour mesurer la concentration de sel indiquée par l'électrode (13).

11. Procédé de fonctionnement d'un dispositif portable pour analyser un contenu de sel dans un aliment selon la revendication 10, comprenant, pour initialiser le dispositif pour analyser le contenu de sel :
opérer l'injecteur de solution de calibrage (24) pour introduire une solution de calibrage à concentration de sel prédéterminée dans le récipient et opérer lesdits moyens moteurs pour mélanger les contenus du récipient ;
mesurer la concentration de sel indiquée par l'électrode ;
calculer un calibrage de ladite électrode en tant que fonction de la concentration de sel mesurée et de la concentration de sel prédéterminée de la solution de calibrage ;
en particulier, lorsque l'injecteur de solution de calibrage (24) est opéré, pour introduire la solution de calibrage dans le récipient, opérant l'injecteur d'eau pour introduire de l'eau dans le récipient pour diluer la concentration de solution de calibrage jusqu'à une concentration de sel prédéterminée de la solution de calibrage.

12. Procédé de fonctionnement d'un dispositif portable pour analyser un contenu de sel dans un aliment selon la revendication 10 ou 11 qui, en opérant lesdits moyens moteurs (6) pour broyer les contenus du récipient, comprend, pour introduire un volume d'eau prédéterminé (17) dans le récipient :
opérer préalablement l'injecteur d'eau (24) pour introduire une première partie du volume d'eau prédéterminé dans le récipient avant d'opérer lesdits moyens moteurs ; et
opérer par la suite l'injecteur d'eau (24) pour introduire une seconde partie du volume d'eau prédéterminé dans le récipient après avoir opéré lesdits moyens moteurs.

13. Procédé de fonctionnement d'un dispositif portable pour analyser un contenu de sel dans un aliment selon l'une quelconque des revendications 10-12, comprenant :
consulter une base de données de produits de référence et, en fonction de la concentration de sel mesurée, afficher un classement :
ou afficher un indicateur de couleur variable sur un écran avec la concentration de sel mesurée.

14. Procédé de fonctionnement d'un dispositif portable pour analyser un contenu de sel dans un aliment selon l'une quelconque des revendications 10-13, comprenant ne pas opérer l'injecteur d'eau pour introduire de l'eau dans le récipient jusqu'à ce qu'il détecte que le couvercle est fermé; ou ne pas opérer l'injecteur de solution de calibrage pour introduire la solution dans le récipient jusqu'à ce qu'il détecte que le couvercle est fermé ; ou ne pas opérer lesdits moyens moteurs jusqu'à ce qu'il détecte que le couvercle est fermé ; ou ne pas opérer les moyens moteurs pour broyer jusqu'à ce qu'il détecte que l'électrode n'est pas accouplée au couvercle ; ou ne pas opérer les moyens moteurs pour mélanger jusqu'à ce qu'il détecte que l'électrode est accouplée au couvercle, ou des combinaisons de ceux-ci.

15. Procédé de fonctionnement d'un dispositif portable pour analyser un contenu de sel dans un aliment selon l'une quelconque des revendications 10-14, comprenant accéder à la base de données et stocker les données de la mesure réalisée comprenant la concentration de sel mesurée.
